# EUROPEAN PATENT SPECIFICATION

(11) **EP 1 638 468 B1**
(45) Date of publication and mention of the grant of the patent: **15.08.2007**
(21) Application number: 04756456.2
(22) Date of filing: 29.06.2004
(51) Int. Cl.: A61B 17/20, A61M 37/00

(54) **METHOD FOR COATING SKIN PIERCING MICROPROJECTIONS**
VERFAHREN ZUM BESCHICHTEN VON MIKROVORSPRÜNGEN ZUM DURCHSTECHEN DER HAUT
METHODE D'ENDUCTION DE MICROPROJECTIONS DE PER AGE DE LA PEAU

(30) Priority: 30.06.2003 US 484142 P
(43) Date of publication of application: 29.03.2006
(73) Proprietor: Alza Corporation, Mountain View, CA 94039-7210 (US)
(72) Inventor: DADDONA, Peter, Menlo Park, CA 94025 (US); CHAN, Keith, T., Sunnyvale, CA 94086 (US); DONNER, John, W., Portola Valley, CA 94028 (US); AYER, Rupal, Cupertino, CA 95014 (US)
(74) Representative: Williams, Paul Edwin
(86) International application number: PCT/US2004/021076
(87) International publication number: WO 2005/004729

(56) References cited:
- US-A1- 2002 138 049
- US-A1- 2002 177 839

## Description

### FIELD OF THE PRESENT INVENTION

This invention relates to a method of coating skin piercing microprojections in order to provide for transdermal delivery of an agent into or through-the skin. More particularly, the invention relates to a method of making a percutaneous drug delivery system for administering a therapeutically active agent (e.g., a drug or vaccine) into or through the skin using skin piercing microprojections which have a dry coating of the agent precisely located on predetermined areas of the microprojections. Delivery of the agent is achieved when the microprojections pierce the skin of a patient and the patient's interstitial fluid contacts and dissolves the active agent.

### BACKGROUND OF THE INVENTION

Transdermal drug delivery systems generally rely on passive diffusion to administer the drug, while active transdermal drug delivery systems rely on an external energy source (e.g., electricity, sonic energy, or heat) to deliver the drug. Passive transdermal drug delivery systems are more common. Passive transdermal systems typically include a drug reservoir which contains a high concentration of drug and is adapted to contact the skin where the drug diffuses through the skin and into the body tissues or bloodstream of a patient. The transdermal drug flux is dependent upon the condition of the skin, the size and physical/chemical properties of the drug molecule, presence of various permeation enhancers and the concentration gradient across the skin. Because of the low skin permeability to many drugs, transdermal delivery has had limited applications. This low permeability is attributed primarily to the stratum corneum, the outermost skin layer which consists of flat, dead cells filled with keratin fibers (keratinocytes) surrounded by lipid bilayers. The highly-ordered structure of the lipid bilayers confers a relatively impermeable character to the stratum corneum.

One method of increasing the passive transdermal diffusional drug flux involves mechanically penetrating or disrupting the outermost skin layers and thereby creating pathways into the skin in order to enhance the amount of agent being transdermally delivered. Early vaccination devices known as scarifiers generally included a plurality of tines or needles which are applied to the skin to scratch or make small cuts in the area of application. The vaccine was applied either topically on the skin, as disclosed in Rabenau, U.S. Patent 5,487,726, or as a wetted liquid applied to the scarifier tines, as disclosed in Galy, U.S. Patent 4,453,926, Chacornac, U.S. Patent 4,109,655, and Kravitz, U.S. Patent 3,136,314, or as a dry coating on and between the scarifier tines, as disclosed in Kravitz, US Patent 3,351,059. Scarifiers have been suggested for intradermal vaccine delivery, in part, because only very small amounts of the vaccine need to be delivered into the skin to be effective in immunizing the patient. Further, the amount of vaccine delivered is not particularly critical since an excess amount achieves satisfactory immunization as well as a minimum amount.

However, a serious disadvantage in using a scarifier to deliver a drug is the difficulty in determining the transdermal drug flux and the resulting dosage delivered. Also, due to the elastic, deforming and resilient nature of skin to deflect and resist puncturing, very tiny (e.g., having lengths less than about 0.5 mm) skin piercing elements often do not uniformly penetrate the skin and/or are wiped free of a coating, particularly a liquid coating, of an agent upon skin penetration. Additionally, due to the self healing process of the skin, the punctures or slits made in the skin tend to close up after removal of the piercing elements from the stratum corneum, Thus, the elastic nature of the skin acts to remove active agent coating the tiny piercing elements upon penetration. Furthermore, the tiny slits formed by the piercing elements heal quickly after removal of the device, thus, limiting the passage of agent through the passageways created by the piercing elements and in turn limiting the transdermal flux of such devices.

Other devices which use tiny skin piercing elements to enhance transdermal drug delivery are disclosed in European Patent EP 0407063A1, Godshall, et al. US Patent 5,879,326; Ganderton, et al. US Patent 3,814,097; Gross, et al. US Patent 5,279,544; Lee, et al. US Patent 5,250,023; Gerstel, et al. US Patent 3,964,482; Kravitz, et al. US Patent Reissue 25,637 and PCT Publication Nos. WO 96/37155, WO 96/37256, WO 96/17648, WO 97/03718, WO 98/11937, WO 98/00193, WO 97/48440, WO 97/48441, WO 97/48442, WO 98/00193, WO 99/64580, WO 98/28037, WO 98/29298, and WO 98/29365. These devices use piercing elements of various shapes and sizes to pierce the outermost layer (i.e., the stratum corneum) of the skin. The piercing elements disclosed in these references generally extend perpendicularly from a thin, flat member, such as a pad or sheet. The piercing elements in some of these devices are extremely small, some having dimensions (i.e., a microprojection length and width) of only about 25 - 400 µm and a microprojection thickness of only about 5 - 50 µm. These tiny piercing/cutting elements make correspondingly small microslits/microcuts in the stratum corneum for enhanced transdermal agent delivery therethrough.

More recently, Cormier et al., in US Patent Application Serial No. 10/045,842, filed October 26,2001, disclose a device for transdermally delivering a potent drug. The device has a plurality of skin piercing microprojections which have a dry coating of the drug. Cormier et al. disclose microfluidic coating techniques, such as ink jet printing, to selectively coat the drug only on the skin piercing microprojections rather than on other portions/surfaces of the device which do not penetrate into the skin. In spite of these disclosures, Cormier et al. do not address the difficulties in aiming the microfluidic spray or depositing the coating only onto the portions of the device which pierce the skin. Thus, there is a need for a precisely controlled coating method which can reproducibly coat only predetermined areas of the microprojections, which typically would include skin-piercing portions of the microprojections.

Trautman and Wright have disclosed a method and device of coating microblades. (Serial No. 10/099,604 filed on 15 March 2002). This application discloses several embodiments of methods and devices for controlling very precisely the thickness of a layer of a coating formulation and then dipping the finalized formed microprojection arrays through this controlled layer of formulation. These procedures require that the microprojections have already been bent out of and generally perpendicular to the sheet which was used to etch or punch out the microprojections.
US Patent Application No. 200/0177839 A1, from which claims 1 and 20 eminate, discloses a transdermal delivery device which comprises at least one stratum corneum-piercing microprojection. The microprojection has a portion with a hydrophilic coating comprising a biologically active agent.

Accordingly, it is an object of the invention to provide a coating having a biologically active agent on a microprojection.

It is a further object of the invention to reliably coat desired areas of a microprojection.

It is yet another object of the invention to minimize the amount of biologically active agent-used to coat a-microprojection by controlling which portions of the microprojection are covered.
These objects are achieved by a transdermal delivery device according to claim 1 and by a method for forming this device according to claim 20. Preferred embodiments are set forth in the dependent claims.

### SUMMARY OF THE INVENTION

The method of the present invention overcomes the difficulties of the prior art by providing a means of applying a liquid coating to precisely controlled areas and locations on the microprojections. The method of the present invention is useful for coating a liquid onto a microprojection with the area of the coating controlled to a precise location. The present invention also provides for the subsequent drying of the above liquid coatings in order to form a dried coating located on predetermined and precise locations on the one or more microprojections of a formed microprojection array.

Initially the microprojections are etched from or punched out of a sheet, preferably a metal sheet, wherein the microproj ections are created by etching or punching openings through the sheet. At this stage the sheet will be referred to as the "etched sheet". The microprojections are then folded or bent out of the plane of the sheet. After the microprojections have been bent, the sheet will be referred to as the "formed microprojection array".

The method includes providing an agent-containing hydrophilic coating liquid and conveying the liquid onto an etched sheet onto which a hydrophobic coating mask has been applied. This hydrophobic coating mask covers all areas of the etched sheet, except the areas onto which the hydrophilic agent-containing liquid is to be located. An important advantage of the present invention is that for very expensive or rare agents (e.g., drugs or vaccines), the agent is coated only on those portions of the device which pierce into the skin, i.e., only the microprojections and not other parts of the etched sheet are coated with the agent. Because of the precise manner in which the hydrophilic coating can be located, various configurations of the agent coating can selected in order to achieve desired engineering and design goals.

A preferred embodiment of the invention is transdermal delivery device for delivering a biologically active agent comprising at least one stratum corneum-piercing microprojection, wherein the microprojection has a first portion with a hydrophobic coating and a second portion with a hydrophilic coating comprising the biologically active agent. Preferably, the second portion comprises a distal portion of the microprojection.

Alternatively, the transdermal delivery device for delivering a biologically active agent comprises a microprojection array of a plurality of stratum corneum-piercing microprojections, wherein at least a portion of the microprojections has a hydrophilic coating comprising the biologically active agent and wherein a portion of the device has a hydrophobic coating. The hydrophilic coating can be applied to a distal portion of the microprojection or to substantially all of the microprojection.

In each of the noted embodiments, the microprojection is configured to pierce through the stratum corneum to a depth of less than about 500 micrometers. Preferably, the microprojection has a length of less than about 500 micrometers and a thickness of less than about 25 micrometers. Also preferably, the hydrophilic coating has a thickness equal to or less than the thickness of the microprojection.

In one embodiment, the stratum corneum-piercing microprojection is formed by etching the microprotrusion from a thin sheet and folding the microprojection out of a plane of the sheet. The thin sheet can comprise a metallic material, such as stainless steel, titanium, nickel titanium alloys and other biocompatible metals.

In another embodiment of the invention, the hydrophobic coating is applied to the microprojection using photolithography. In the noted embodiments, the hydrophobic coatings is disposed on a portion of the microprojection having been washed free of unexposed photoresist and the hydrophilic coating is disposed on a portion of the microprojection having been washed free of solubilized exposed photoresist.

In some embodiments of the invention, the hydrophilic coating has a viscosity less than about 0,5 Pas (500 centipoise).

In further embodiments of the invention, the device comprises multiple microprojections having the hydrophobic coating and the hydrophilic coating. Preferably, such embodiments have a density of at least about 10 microprojections/cm2 and more preferably of about 200 - 2000 microprojections/cm2.

The invention also comprises methods for forming such devices including the steps of forming at least one stratum corneum-piercing microprojection in a thin sheet of material, applying a hydrophobic coating to a first portion of the microprojection, bending the microprojection out of a plane formed by the thin sheet; and applying a hydrophilic coating comprising the biologically active agent to a second portion of the microprojection. The microprojection can be formed by etching or punching, for example. Generally, the hydrophobic coating is removed from the second portion of the microprojection member before applying the hydrophilic coating.

In the noted embodiments, the hydrophobic coating is removed from the second portion of the microprojection member by vaporizing the hydrophobic coating with radiation. Alternatively, the hydrophobic coating is removed from the second portion of the microprojection by contacting the portion with a micro-stamp.

In other embodiments of the invention, the step of applying a hydrophobic coating to a first portion of the microprojection comprises coating the microprojection with photoresist, masking the second portion of the microprojection, exposing the microprojection to radiation, washing unexposed photoresist from the first portion of the microprojection member, applying the hydrophobic coating to the first and second -portions of the microprojection, solubilizing exposed photoresist on the second portion of the microprojection, and washing the solubilized exposed photoresist from the second portion of the microprojection.

In yet other embodiments of the invention, the transdermal delivery device is formed by providing a device having at least one microprojection configured to pierce the stratum corneum, applying a photoresist to the device, masking the device so that a first portion of the microprojection is uncovered, exposing the masked device to radiation, washing unexposed photoresist from the device, applying a hydrophobic coating to the device, solubilizing exposed photoresist on the first portion, washing solubilized photoresist from the first portion to remove the hydrophobic coating, and applying a hydrophilic coating comprising the biologically active agent to the first portion of the microprojection.

The biologically active agent for coating the microprojections is preferably selected to have sufficient potency to be therapeutically effective when administered transdermally in an amount of less than about 1 mg, and more preferably less than about 0.25 mg, of active agent. The most preferred agents are vaccines and potent drugs.

Such agents include therapeutic agents in all the major therapeutic areas including, but not limited to, anti-infectives, such as antibiotics and antiviral agents; analgesics, including fentanyl, sufentanil, remifentanil, buprenorphine and analgesic combinations; anesthetics; anorexics; antiarthritics; antiasthmatic agents such as terbutaline; anticonvulsants; antidepressants; antidiabetic agents; antidiarrheals; antihistamines; anti-inflammatory agents; antimigraine preparations; antimotion sickness preparations such as scopolamine and ondansetron; antinauseants; antineoplastics ; antiparkinsonism drugs; antipruritics; antipsychotics; antipyretics; antispasmodics, including gastrointestinal and urinary; anticholinergics; sympathomimetrics; xanthine derivatives; cardiovascular preparations, including calcium channel blockers such as nifedipine; beta blockers; beta-agonists such as dobutamine and ritodrine; antiarrythmics; antihypertensives such as atenolol; ACE inhibitors such as ranitidine; diuretics; vasodilators, including general, coronary, peripheral, and cerebral; central nervous system stimulants; cough and cold preparations; decongestants; diagnostics; hormones such as parathyroid hormone; hypnotics; immunosuppressants; muscle relaxants; parasympatholytics; parasympathomimetrics; prostaglandins; proteins; peptides; psychostimulants; sedatives; and tranquilizers. Other suitable agents include vasoconstrictors, anti-healing agents and pathway patency modulators.

Further specific examples of agents include, without limitation, growth hormone release hormone (GHRH), growth hormone release factor (GHRF), insulin, insultropin, calcitonin, octreotide, endorphin, TRN, NT-36 (chemical name: N-[[(s)-4-oxo-2-azetidinyl] carbonyl]-L-histidyl-L-prolinamide), liprecin, pituitary hormones (e.g., HGH, HMG, desmopressin acetate, etc), follicle luteoids, aANF, growth factors such as growth factor releasing factor (GFRF), bMSH, GH, somatostatin, bradykinin, somatotropin, platelet-derived growth factor releasing factor, asparaginase, bleomycin sulfate, chymopapain, cholecystokinin, chorionic gonadotropin, erythropoietin, epoprostenol (platelet aggregation inhibitor), gluagon, HCG, hirulog, hyaluronidase, interferon alpha, interferon beta, interferon gamma, interleukins, interleukin-10 (IL-10), erythropoietin (EPO), granulocyte macrophage colony stimulating factor (GM-CSF), granulocyte colony stimulating factor (G-CSF), glucagon, leutinizing hormone releasing hormone (LHRH), LHRH analogs (such as goserelin, leuprolide, buserelin, triptorelin, gonadorelin, and napfarelin, menotropins (urofollitropin (FSH) and LH)), oxytocin, streptokinase, tissue plasminogen activator, urokinase, vasopressin, deamino [Va14, D-Arg8] arginine vasopressin, desmopressin, corticotropin (ACTH), ACTH analogs such as ACTH (1-24), ANP, ANP clearance inhibitors, angiotensin II antagonists, antidiuretic hormone agonists, bradykinn antagonists, ceredase, CSI's, calcitonin gene related peptide (CGRP), enkephalins, FAB fragments, IgE peptide suppressors, IGF-1, neurotrophic factors, colony stimulating factors, parathyroid hormone and agonists, parathyroid hormone antagonists, parathyroid hormone (PTH), PTH analogs such as PTH (1-34), prostaglandin antagonists, pentigetide, protein C, protein S, renin inhibitors, thymosin alpha-1, thrombolytics, TNF, vasopressin antagonists analogs, alpha-1 antitrypsin (recombinant), and TGF-beta.

The noted biologically active agents can also be in various forms, such as free bases, acids, charged or uncharged molecules, components of molecular complexes or nonirritating, pharmacologically acceptable salts. Further, simple derivatives of the active agents (such as ethers, esters, amides, etc.), which are easily hydrolyzed at body pH, enzymes, etc., can be employed.

In one embodiment of the invention, the microprojection member has a cm² microprojection density of at least approximately 10 microprojections/cm², more preferably, in the range of at least approximately 200 - 2000 microprojections/cm².

In one embodiment, the microprojection member is constructed out of stainless steel, titanium, nickel titanium alloys, or similar biocompatible materials.

In another embodiment, the microprojection member is constructed out of a non conductive material. Alternatively, the microprojection member can be coated with a non-conductive material, such as Parylene®, or a hydrophobic material, such as Teflon®, silicon or other low energy material.

The coating formulations applied to the microprojection member to form solid biocompatible coatings can comprise aqueous and non-aqueous formulations having at least one agent, which can be dissolved within a biocompatible carrier or suspended within the carrier.

In certain embodiments of the invention, the viscosity of a biologically active agent formulation for coating microprojections is enhanced by adding low volatility counterions. In one embodiment, the agent has a positive charge at the formulation pH and the viscosity-enhancing counterion comprises an acid having at least two acidic pKas. Suitable acids include maleic acid, malic acid, malonic acid, tartaric acid, adipic acid, citraconic acid, fumaric acid, glutaric acid, itaconic acid, meglutol, mesaconic acid, succinic acid, citramalic acid, tartronic acid, citric acid, tricarballylic acid, ethylenediaminetetraacetic acid, aspartic acid, glutamic acid, carbonic acid, sulfuric acid, and phosphoric acid.

Another preferred embodiment is directed to a viscosity-enhancing mixture of counterions wherein the agent has a positive charge at the formulation pH and at least one of the counterion is an acid having at least two acidic pKas. The other counterion is an acid with one or more pKas. Examples of suitable acids include hydrochloric acid, hydrobromic acid, nitric acid, sulfuric acid, maleic acid, phosphoric acid, benzene sulfonic acid, methane sulfonic acid, citric acid, succinic acid, glycolic acid, gluconic acid, glucuronic acid, lactic acid, malic acid, pyruvic acid, tartaric acid, tartronic acid, fumaric acid, acetic acid, propionic acid, pentanoic acid, carbonic acid, malonic acid, adipic acid, citraconic acid, levulinic acid, glutaric acid, itaconic acid, meglutol, mesaconic acid, citramalic acid, citric acid, aspartic acid, glutamic acid, tricarballylic acid and ethylenediaminetetraacetic acid.

Generally, in the noted embodiments of the invention, the amount of counterion should neutralize the charge of the biologically active agent. In such embodiments, the counterion or the mixture of counterion is present in amounts necessary to neutralize the charge present on the agent at the pH of the formulation.-Excess of counterion (as the free acid or as a salt) can be added to the peptide in order to control pH and to provide adequate buffering capacity.

In another preferred embodiment, the agent has a positive charge and the counterion is a viscosity-enhancing mixture of counterions chosen from the group of citric acid, tartaric acid, malic acid, hydrochloric acid, glycolic acid, and acetic acid. Preferably, counterions are added to the formulation to achieve a viscosity in the range of about 2.10⁻² - 0,2 Pas (20 - 200 cp).

In a preferred embodiment, the viscosity-enhancing counterion is an acidic counterion such as a low volatility weak acid. Low volatility weak acid counterions present at least one acidic pKa and a melting point higher than about 50°C or a boiling point higher than about 170°C at Pₐₜₘ. Examples of such acids include citric acid, succinic acid, glycolic acid, gluconic acid, glucuronic acid, lactic acid, malic acid, pyruvic acid, tartaric acid, tartronic acid, and fumaric acid.

In another preferred embodiment the counterion is a strong acid. Strong acids can be defined as presenting at least one pKa lower than about 2. Examples of such acids include hydrochloric acid, hydrobromic acid, nitric acid, sulfonic acid, sulfuric acid, maleic acid, phosphoric acid, benzene sulfonic acid and methane sulfonic acid.

Another preferred embodiment is directed to a mixture of counterions wherein at least one of the counterion is a strong acid and at least one of the counterion is a low volatility weak acid.

Another preferred embodiment is directed to a mixture of counterions wherein at least one of the counterion is a strong acid and at least one of the counterion is a weak acid with high volatility. Volatile weak acid counterions present at least one pKa higher than about 2 and a melting point lower than about 50°C or a boiling point lower than about 170°C at Pₐₜₘ. Examples of such acids include acetic acid, propionic acid, pentanoic acid and the like.

The acidic counterion is present in amounts necessary to neutralize the positive charge present on the drug at the pH of the formulation. Excess of counterion (as the free acid or as a salt) can be added to the drug in order to control pH and to provide adequate buffering capacity.

In another embodiment of the invention, the coating formulation includes at least one buffer. Examples of such buffers include ascorbic acid, citric acid, succinic acid; glycolic acid, gluconic acid, glucuronic acid, lactic acid, malic acid, pyruvic acid, tartaric acid, tartronic acid, fumaric acid, maleic acid, phosphoric acid, tricarballylic acid, malonic acid, adipic acid, citraconic acid, glutaratic acid, itaconic acid, mesaconic acid, citramalic acid, dimethylolpropionic acid, tiglic acid, glyceric acid, methacrylic acid, isocrotonic acid, b-hydroxybutyric acid, crotonic acid, angelic acid, hydracrylic acid, aspartic acid, glutamic acid, glycine or mixtures thereof.

In one embodiment of the invention, the coating formulations include at least one antioxidant, which can be sequestering agents such sodium citrate, citric acid, EDTA (ethylene-dinitrilo-tetraacetic acid) or free radical scavengers, such as ascorbic acid, methionine, sodium ascorbate, and the like.

In one embodiment of the invention, the coating formulation includes at least one surfactant, which can be zwitterionic, amphoteric, cationic, anionic, or nonionic, including, without limitation, sodium lauroamphoacetate, sodium dodecyl sulfate (SDS), cetylpyridinium chloride (CPC), dodecyltrimethyl ammonium chloride (TMAC), benzalkonium, chloride, polysorbates such as Tween 20 and Tween 80, other sorbitan derivatives, such as sorbitan laurate, and alkoxylated alcohols, such as laureth-4.

In a further embodiment of the invention, the coating formulation includes at least one polymeric material or polymer that has amphiphilic properties, which can comprise, without limitation, cellulose derivatives, such as hydroxyethylcellulose (HEC), hydroxypropylmethylcellulose (HPMC), hydroxypropycellulose (HPC), methylcellulose (MC), hydroxyethylmethylcellulose (HEMC), or ethylhydroxy ethylcellulose (EHEC), as well as pluronics.

In another embodiment, the coating formulation includes a hydrophilic polymer selected from the following group: hydroxyethyl starch, dextran, poly(vinyl alcohol), poly(ethylene oxide), poly(2-hydroxyethylmethacrylate), poly(n-vinyl pyrolidone), polyethylene glycol and mixtures thereof , and like polymers.

In another embodiment of the invention, the coating formulation includes a biocompatible carrier, which can comprise, without limitation, human albumin, bioengineered human albumin, polyglutamic acid, polyaspartic acid, polyhistidine, pentosan polysulfate, polyamino acids, sucrose, trehalose, melezitose, raffinose and stachyose.

In another embodiment, the coating formulation includes a stabilizing agent, which can comprise, without limitation, a non-reducing sugar, a polysaccharide or a reducing sugar. Suitable non-reducing sugars for use in the methods and compositions of the invention include, for example, sucrose, trehalose, stachyose, or raffinose. Suitable polysaccharides for use in the methods and compositions of the invention include, for example, dextran, soluble starch, dextrin, and insulin. Suitable reducing sugars for use in the methods and compositions of the invention include, for example, monosaccharides such as, for example, apiose, arabinose, lyxose, ribose, xylose, digitoxose, fucose, quercitol, quinovose, rhamnose, allose, altrose, fructose, galactose, glucose, gulose, hamamelose, idose, mannose, tagatose, and the like; and disaccharides such as, for example, primeverose, vicianose, rutinose, scillabiose, cellobiose, gentiobiose, lactose, lactulose, maltose, melibiose, sophorose, and turanose, and the like.

In another embodiment, the coating formulation includes a vasoconstrictor, which can comprise, without limitation, amidephrine, cafaminol, cyclopentamine, deoxyepinephrine, epinephrine, felypressin, indanazoline, metizoline, midodrine, naphazoline, nordefrin, octodrine, ornipressin, oxymethazoline, phenylephrine, phenylethanolamine, phenylpropanolamine, propylhexedrine, pseudoephedrine, tetrahydrozoline, tramazoline, tuaminoheptane, tymazoline, vasopressin, xylometazoline and the mixtures thereof. The most preferred vasoconstrictors include epinephrine, naphazoline, tetrahydrozoline indanazoline, metizoline, tramazoline, tymazoline, oxymetazoline and xylometazoline.

In another embodiment of the invention, the coating formulation includes at least one "pathway patency modulator", which can comprise, without limitation, osmotic agents (e.g., sodium chloride), zwitterionic compounds (e.g., amino acids), and anti-inflammatory agents, such as betamethasone 21-phosphate disodium salt, -triamcinolone acetonide 21 disodium phosphate, hydrocortamate hydrochloride, hydrocortisone 21-phosphate disodium salt, methylprednisolone 21-phosphate disodium salt, methylprednisolone 21-succinaate sodium salt, paramethasone disodium phosphate and prednisolone 21-succinate sodium salt, and anticoagulants, such as citric acid, citrate salts (e.g., sodium citrate), dextrin sulfate sodium, aspirin and EDTA.

In yet another embodiment of the invention, the coating formulation includes a solubilising/complexing agent, which can comprise Alpha-Cyclodextrin, Beta-Cyclodextrin, Gamma-Cyclodextrin, glucosyl-alpha-Cyclodextrin, maltosyl-alpha-Cyclodextrin, glucosyl-beta-Cyclodextrin, maltosyl-beta-Cyclodextrin, hydroxypropyl beta-cyclodextrin, 2-hydroxypropyl-beta-Cyclodextrin, 2-hydroxypropyl-gamma-Cyclodextrin, hydroxyethyl-beta-Cyclodextrin, methyl-beta-Cyclodextrin, sulfobutylether-alpha-cyclodextrin, sulfobutylether-beta-cyclodextrin, and sulfobutylether-gamma-cyclodextrin. Most preferred solubilising/complexing agents are beta-cyclodextrin, hydroxypropyl beta-cyclodextrin, 2-hydroxypropyl-beta-Cyclodextrin and sulfobutylether7 beta-cyclodextrin.

In another embodiment of the invention, the coating formulation includes at least one non-aqueous solvent, such as ethanol, isopropanol, methanol, propanol, butanol, propylene glycol, dimethysulfoxide, glycerin, N,N-dimethylformamide and polyethylene glycol 400.

Preferably, the coating formulations have a viscosity less than approximately 0,5 Pas (500 centipoise) and greater than 3.10⁻³ Pas (3 centipose)

In one embodiment of the invention, the thickness of the biocompatible coating is less than 25 microns, more preferably, less than 10 microns, as measured from the microprojection surface.

### BRIEF DESCRIPTION OF THE DRAWINGS

The invention will now be described in greater detail with reference to the preferred embodiments illustrated in the accompanying drawings and figures. wherein:

FIGURE 1A is a perspective view of a portion of one example of a formed microprojection array;

FIGURE 1B is a perspective view of the formed microprojection array of FIG. 1 with a coating deposited onto the microprojections;

FIGURES 2-8 are schematic representations of the registered mask coating method of the invention wherein the steps of the method are described as follows:

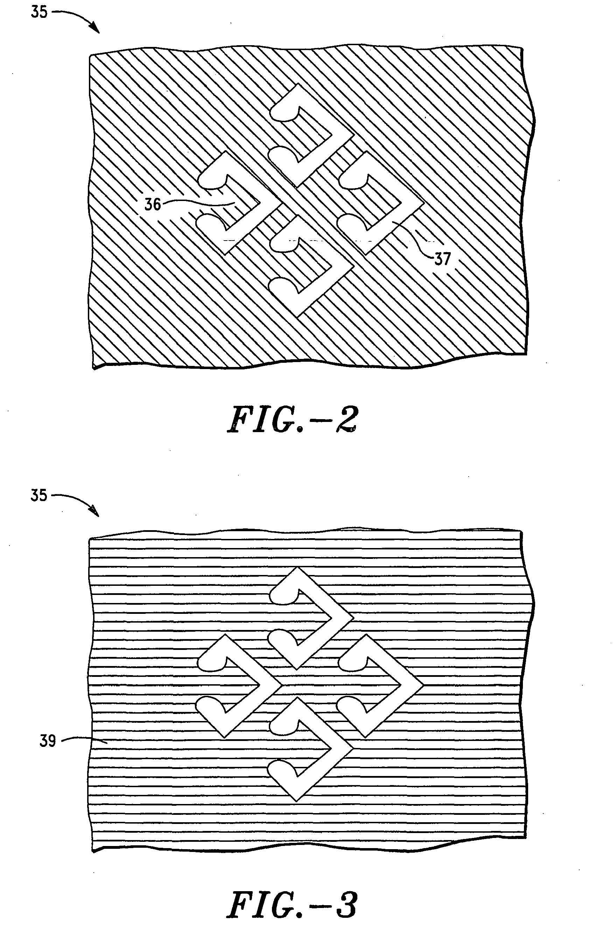

FIGURE 2 shows an etched sheet which has already had the microprojections created; all of the surfaces shown are bare metal which forms the array;

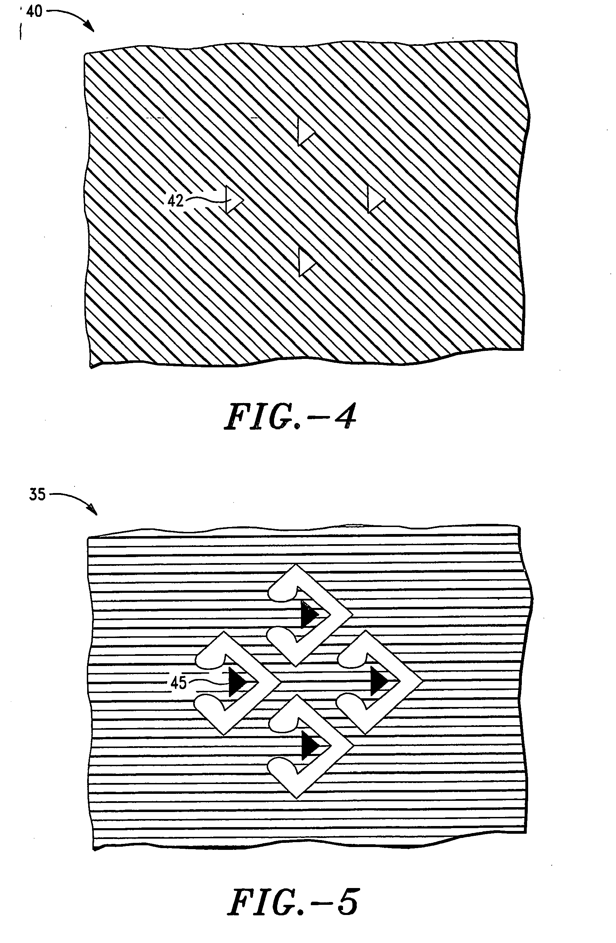

FIGURE 3 shows the etched sheet of FIGURE 2 after having been completely coated with a photosensitive resist;

FIGURE 4 shows the photomask;

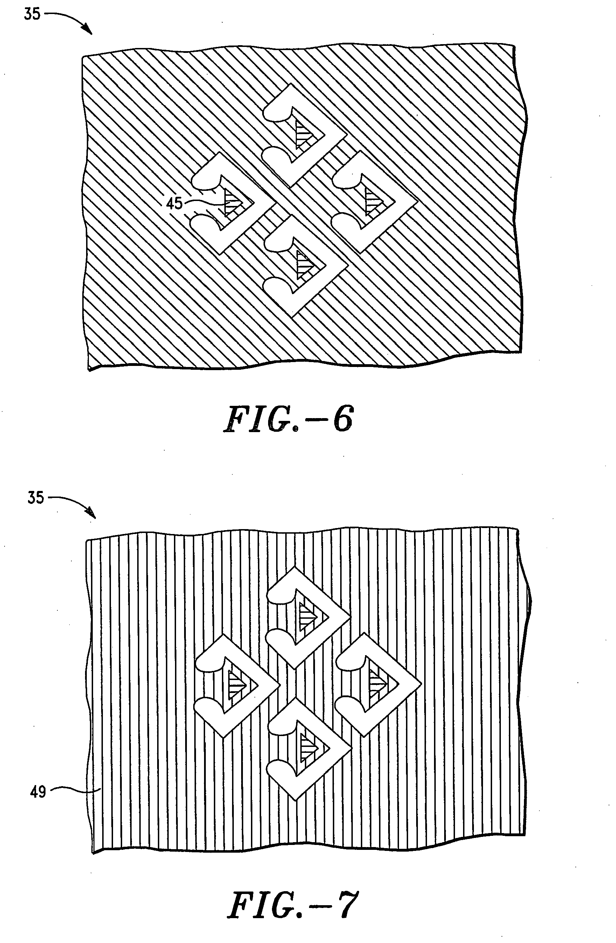

FIGURE 5 shows the etched sheet of FIGURE 3 after having been exposed to light through the mask;

FIGURE 6 shows the etched sheet of FIGURE 5 after the portions of the resist which weren't exposed to light have been removed;

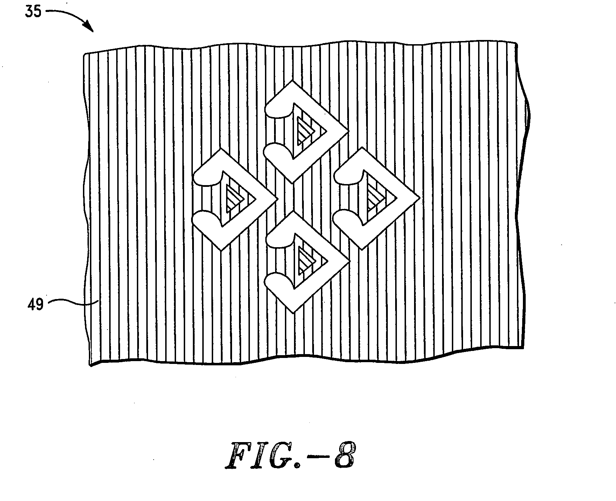

FIGURE 7 shows the etched sheet of FIGURE 6 after having been coated with a hydrophobic second layer, which covers the formed microprojection array everywhere except those portions that were exposed to light through the mask;

FIGURE 8 shows the etched sheet of FIGURE 7 after remaining areas of the resist layer have been removed.

### DETAILED DESCRIPTION OF THE INVENTION Definitions

The term "transdermal" means the delivery of an agent (e.g., a drug or vaccine) into and/or through the skin for local or systemic therapy.

The term "transdermal flux" means the rate of transdermal delivery.

The term "microprojections" refers to piercing elements which are adapted to pierce or cut through the stratum corneum into the underlying epidermis layer, or epidermis and dermis layers, of the skin of a living animal, particularly a human. The piercing elements should not pierce the skin to a depth that causes significant bleeding. Typically the piercing elements have a blade length of less than 500 µm, and preferably less than 400 µm. The microprojections typically have a width of about 75 to 500 µm and a thickness of about 5 to 50 µm. The microprojections may be formed in different shapes, such as needles, hollow needles, blades, pins, punches, and combinations thereof. As such, the terms "microprojections," "microprotrusions," "microblades" and "microneedles" are used throughout interchangeably.

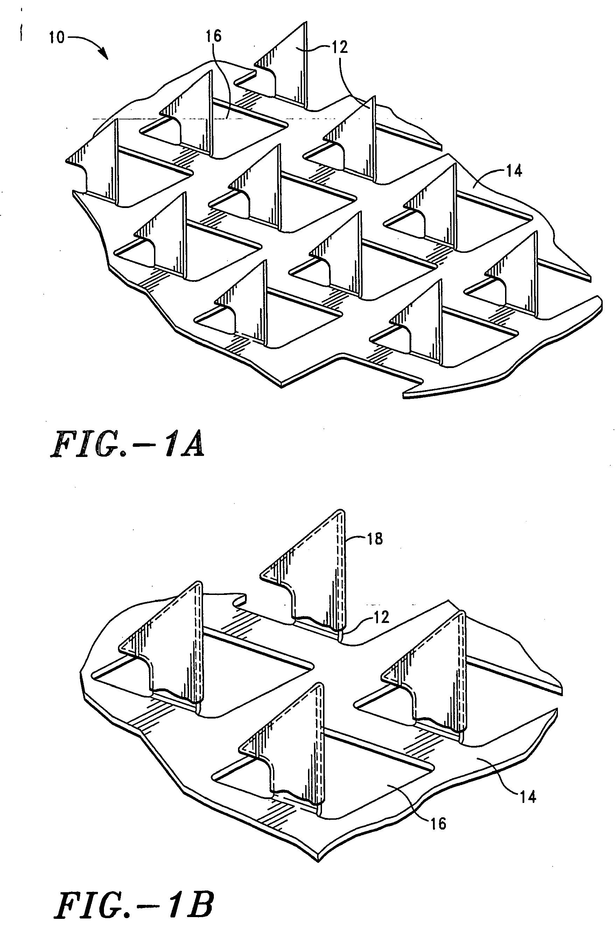

The term "microprojection array" as used herein refers to a plurality of microprojections arranged in an array for piercing the stratum corneum. The microprojection array may be created by etching or punching a plurality of microprojections from a thin sheet and folding or bending the microprojections out of the plane of the sheet to form a configuration such as that shown in FIG. 1A. The microprojection array may also be fabricated in other known manners, such as by fabricating one or more strips having microprojections along an edge of each of the strip(s), as disclosed in Zuck, US Patent 6,050,988.

The term "biologically active agent" comprises any agent or drug having therapeutic or biological effect. Such agents include agents in all the major therapeutic areas including, but not limited to: anti-infectives such as antibiotics and antiviral agents; analgesics, including fentanyl, sufentanil, remifentanil, buprenorphine and analgesic combinations; anesthetics; anorexics; antiarthritics; antiasthmatic agents such as terbutaline; anticonvulsants; antidepressants; antidiabetic agents; antidiarrheals; antihistamines; anti-inflammatory agents; antimigraine preparations; antimotion sickness preparations such as scopolamine and ondansetron; antinauseants; antineoplastics ; antiparkinsonism drugs; antipruritics; antipsychotics; antipyretics; antispasmodics, including gastrointestinal and urinary; anticholinergics; sympathomimetrics; xanthine derivatives; cardiovascular preparations, including calcium channel blockers such as nifedipine; beta blockers; beta-agonists such as dobutamine and ritodrine; antiarrythmics; antihypertensives such as atenolol; ACE inhibitors such as ranitidine; diuretics; vasodilators, including general, coronary, peripheral, and cerebral; central nervous system stimulants; cough and cold preparations; decongestants; diagnostics; hormones such as parathyroid hormone; hypnotics; immunosuppressants; muscle relaxants; parasympatholytics; parasympathomimetrics; prostaglandins; proteins; peptides; psychostimulants; sedatives; and tranquilizers. Other suitable agents include vasoconstrictors, anti-healing agents and pathway patency modulators.

Further specific examples of agents include, without limitation, growth hormone release hormone (GHRH), growth hormone release factor (GHRF), insulin, insultropin, calcitonin, octreotide, endorphin, TRN, NT-36 (chemical name: N-[[(s)-4-oxo-2-azetidinyl] carbonyl]-L-histidyl-L-prolinamide), liprecin, pituitary hormones (e.g., HGH, HMG, desmopressin acetate, etc), follicle luteoids, aANF, growth factors such as growth factor releasing factor (GFRF), bMSH, GH, somatostatin, bradykinin, somatotropin, platelet-derived growth factor releasing factor, asparaginase, bleomycin sulfate, chymopapain, cholecystokinin, chorionic gonadotropin, erythropoietin, epoprostenol (platelet aggregation inhibitor), gluagon, HCG, hirulog, hyaluronidase, interferon alpha, interferon beta, interferon gamma, interleukins, interleukin-10 (IL-10), erythropoietin (EPO), granulocyte macrophage colony stimulating factor (GM-CSF), granulocyte colony stimulating factor (G-CSF), glucagon, leutinizing hormone releasing hormone (LHRH), LHRH analogs (such as goserelin, leuprolide, buserelin, triptorelin, gonadorelin, and napfarelin, menotropins (urofollitropin (FSH) and LH)), oxytocin, streptokinase, tissue plasminogen activator, urokinase, vasopressin, deamino [Va14, D-Arg8] arginine vasopressin, desmopressin, corticotropin (ACTH), ACTH analogs such as ACTH (1-24), ANP, ANP clearance inhibitors, angiotensin II antagonists, antidiuretic hormone agonists, bradykinn antagonists, ceredase, CSI's, calcitonin gene related peptide (CGRP), enkephalins, FAB fragments, IgE peptide suppressors, IGF-1, neurotrophic factors, colony stimulating factors, parathyroid hormone and agonists, parathyroid hormone antagonists, parathyroid hormone (PTH), PTH analogs such as PTH (1-34), prostaglandin antagonists, pentigetide, protein C, protein S, renin inhibitors, thymosin alpha-1, thrombolytics, TNF, vasopressin antagonists analogs, alpha-1 antitrypsin (recombinant), and TGF-beta.

The noted biologically active agents can also be in various forms, such as free bases, acids, charged or uncharged molecules, components of molecular complexes or nonirritating, pharmacologically acceptable salts. Further, simple derivatives of the active agents (such as ethers, esters, amides, etc.), which are easily hydrolyzed at body pH, enzymes, etc., can be employed.

The term "biologically effective amount" or "biologically effective rate" shall be used when the biologically active agent is a pharmaceutically active agent and refers to the amount or rate of the pharmacologically active agent needed to effect the desired therapeutic, often beneficial, result. The amount of active agent employed in the hydrogel formulations and coatings of the invention will be that amount necessary to deliver a therapeutically effective amount of the active agent to achieve the desired therapeutic result.

In practice, this will vary widely depending upon the particular biologically active agent being delivered, the site of delivery, the severity of the condition being treated, the desired therapeutic effect and the dissolution and release kinetics for delivery of the agent from the coating into skin tissues.

The present invention provides a method for selectively applying a hydrophilic agent-containing coating onto the skin-piercing portions of a microprojection device. This hydrophilic agent-containing coating could be applied prior to bending the , microprojections down. The disadvantage of this procedure is that the agent containing coating would likely be damaged during the bending process. This damage could be minimized by applying the coating only on one side of the microprojections, but this reduces the total amount of drug loading by half.

The preferred method is to bend the microprojections down from the sheet after the application of the hydrophobic coating mask but before the application of the hydrophilic agent-containing coating.

The microprojections are adapted to pierce through the stratum corneum into the underlying epidermis layer, or epidermis and dermis layers, but do not penetrate so deep as to reach the capillary beds and cause significant bleeding. Typically, the microprojections have a length that allows skin penetration to a depth of up to about 500 µm, and preferably up to about 400 µm. Upon piercing the stratum corneum layer of the skin, the agent-containing coating is dissolved by body fluid (intracellular fluids and extracellular fluids such as interstitial fluid, blood, or mixtures thereof) and released into the skin for local or systemic therapy.

Fig. 1A illustrates one embodiment of a stratum corneum-piercing formed microprojection array member 10 as commonly used in the art. Fig. 1A shows a portion of the member 10 having a plurality of microprojections 12. The microprojections 12 extend at substantially a 90° angle from a sheet 14 having openings 16. In the embodiment of the formed microprojection array member 10 shown in Figs 1A and 1B, the microprojections 12 are formed by etching or punching a plurality of microprojections 12 from a thin metal sheet 14 and bending the microprojections 12 out of a plane of the sheet. Metals such as stainless steel and titanium are preferred. References herein to etched sheets and microprojection arrays made of titanium should be understood to include not only titanium but stainless steel and other metals. Metal microprojection members and methods of making same are disclosed in Trautman et al, U.S. Patent 6,083,196; Zuck, U.S. Patent 6,050,988; and Daddona et al., U.S. Patent 6,091,975.

Other microprojection members that can be used with the present invention are formed by etching silicon using silicon chip etching techniques or by molding plastic using etched micro-molds. Silicon and plastic microprojection members are disclosed in Godshall et al. U.S. Patent 5,879,326.

Fig. 1B illustrates the microprojection member 10 having microprojections 12 having a pharmacologically active agent containing coating 18. The coating 18 may partially or completely cover the microprojections 12.

In accordance with these prior art methods, the agent-containing coating is applied after the microprojections 12 are formed (i.e., etched) and bent out of the plane of metal sheet 14.

The method of the present invention relates to applying surface modifications to the etched sheet in order to control and/or limit the area on the microprojections that will retain an agent-containing formulation. This method requires the application of a hydrophobic coating to the microprojection in areas where the agent-containing coating is not to be applied. There are two general methods to control how this hydrophobic coating is applied.

The first is to apply the hydrophobic coating over the entire etched sheet and then selectively remove the hydrophobic coating from those areas where the agent-containing coating is to be applied. The removal can be accomplished by treating the desired areas of the hydrophobic layer with laser radiation. The laser treatment will literally vaporize the regions of the hydrophobic coating the need to be removed. Because the hydrophobic layer is applied in a relatively thin layer as compared to the underlying metal, it is possible to adjust the power applied by the laser to vaporize the hydrophobic layer without also damaging the underlying metal.

Alternatively, it is possible to use the technique of micro-contact stamping for the removal of the desired areas of the hydrophobic layer. First the etched sheet is completely coated with a hydrophobic layer. Then a micro-stamp, which corresponds to the areas of the hydrophobic layer which need to be removed, is applied. After the application of the micro-stamp, only areas of the etched sheet that are intended to be coated with the hydrophilic agent-containing coating are not covered by the hydrophobic coating mask. This results in a hydrophobic layer coating the etched sheet everywhere but where the agent-containing formulation is to be applied. This technique has been described in "Soft Lithography" by Younan Xi and George M. Whiteside, in Ann. Rev. Mater. Sci. 1998, 28:153-84.

It is also possible to use the above-cited technique of micro-stamping to apply the hydrophobic layer directly to the bare metal of the etched sheet. In this case the micro-stamp corresponds to the areas to which the hydrophobic coating is to be applied. The remaining area of the etched sheet will not be coated and have the bare metal exposed. It is-the exposed bare metal area that will retain the agent-containing coating.

In yet another alternative embodiment, a third method involves a multi-step photolithographic technique utilizing a mask that exposes the areas that are to be coated by the agent-containing formulation.

This embodiment of the method of the present invention is based upon several steps of applying various resists to the titanium sheet which finally results in the titanium having a hydrophobic coating covering most of the titanium sheet as illustrated in Figs. 2-7. The parts of the titanium sheet that are not covered by this coating have the bare metal exposed. The final step is applying an aqueous hydrophilic coating formulation to the sheet. The hydrophobic layer will repel the aqueous solution away from those portions of the sheet that are not intended to be coated. This results in only the bare metal portions of the sheet retaining the coating formulation. After the liquid coating formulation is dried, by one of any number of conventional processes, the metal sheet and the microprojections formed therein are now coated in the specific areas so intended. Typically, the area chosen to be coated is an area near the more distal half of the microprojection. Typically, the piercing edges of the microprojection are not coated with the hydrophilic agent-containing coating so as to not interfere with the cutting action of the piercing leading edges.

Fig. 2 shows the metal microprojection array sheet 35, with the microprojections 36 etched or punched therein. Typically the etching or punching process results in the formation of one or more openings 37 which extend all the way through the microprojection array sheet 35. At this stage, the microprojection array sheet 35 is not coated with any resists or layers.

Fig. 3 shows microprojection array sheet 35 coated with a photoresist layer 39. Photoresist layer 39 is applied completely over the entire surface of microprojection array sheet 35.

Fig. 4 shows mask 40 having mask openings 42 fabricated therein. When mask 40 is carefully placed on top of microprojection array sheet 35, the mask openings 42 will be aligned on top of the areas of the microprojection array sheet 35 that ultimately will be coated with an agent-containing coating. Though the area coated will typically be the distal portions of the microprojections 36, the method of the present invention allows for any area of the microprojection array sheet 35 to ultimately be coated. The microprojection array/mask combination is now exposed to light. The combination is oriented such that the mask is between the photoresist layer 39 and the light source. The specific wavelength, intensity and length of exposure is easily determined by reference to the manufacturer's specifications for the specific photoresist selected. Normally, photoresist layer 39 is soluble in standard solvents. However, those areas of photoresist layer 39 that were exposed to light, are no longer soluble in these standard solvents.

Fig. 5 shows microprojection array sheet 35 after photoresist layer 39 has been exposed to light through mask 40 and mask 40 subsequently having been removed. Those areas that have been exposed to light are shown in black as photoexposed resist 45.

The portions of photoresist layer 39 that have not been exposed to light can be easily washed away by the use of standard solvents and techniques. For this particular embodiment, this washing process results in bare metal over most of the microprojection array sheet 35 (the exposed metal is shown as diagonally hatched in Fig. 6). The areas of the microprojection array sheet 35 which were exposed to light and which resulted in insoluble resist, are shown as horizontally hatched in Fig. 6 and correspond to exposed photoresist 45, shown in Fig. 5.

The next step, shown in Fig. 7, is to coat the microprojection array sheet 35 with a hydrophobic second layer 49. This layer will coat the entire microprojection array including those areas that are still coated by the exposed photoresist 45. This second layer is shown as vertical hatching in Fig. 7. The hydrophobic second layer 49 can be composed of Teflon^{®}, silicone, or other low energy or hydrophobic material.

Then the remaining areas of exposed photoresist are washed away utilizing a special solvent and technique that will solubilize the exposed photoresist 45 and that portion of the overlaying hydrophobic second layer 49 that covers the exposed photoresist 45 areas. As shown in Fig. 8, the final result is a microprojection array sheet 35 largely coated with hydrophobic second layer-49and-smallerc regions of bare hydrophilic metal. At this stage in the method, the smaller regions are on the distal ends of microprojections 36 and are comprised of the exposed metal of the microprojection array sheet 35.

This specially prepared microprojection array sheet 35 can now be exposed to an agent-containing formulation. The hydrophobic second layer 49 will limit the extent of coating by this formulation to those regions of exposed metal on the microprojection array sheet 35.

Once the formulation has been dried onto the microprojection array sheet 35, the result is an array having a dried coating of the agent precisely located on the microprojections.

In all cases, following exposure of the treated microprojection array sheet 35 to the coating formulation, the coating formulation is dried onto the microprojections by various means. In a preferred embodiment the coated device is dried in ambient room conditions. However, various temperatures and humidity levels can be used to dry the coating solution onto the microprojections. Additionally, the devices can be heated, lyophilized, freeze dried or similar techniques used to remove the water from the coating.

At this stage, it is possible to remove the hydrophobic second layer 49, leaving only the agent-containing coating on the microprojections. However, there are advantages to leaving the hydrophobic second layer 49 on the microprojection. Once the microprojection array has been applied to the skin, hydrophobic second layer 49 will minimize the flow of interstitial fluid towards the base of the blade which tends to carry the solubilized agent away from the skin and reduce the amount of agent that is actually absorbed systemically.

In certain embodiments of the invention, the viscosity of a biologically active agent formulation-for-coating-microproj ections-is-enhanced -by -adding-Iew volatility counterions. In one embodiment, the agent has a positive charge at the formulation pH and the viscosity-enhancing counterion comprises an acid having at least two acidic pKas. Suitable acids include maleic acid, malic acid, malonic acid, tartaric acid, adipic acid, citraconic acid, fumaric acid, glutaric acid, itaconic acid, meglutol, mesaconic acid, succinic acid, citramalic acid, tartronic acid, citric acid, tricarballylic acid, ethylenediaminetetraacetic acid, aspartic acid, glutamic acid, carbonic acid, sulfuric acid, and phosphoric acid.

Another preferred embodiment is directed to a viscosity-enhancing mixture of counterions wherein the agent has a positive charge at the formulation pH and at least one of the counterions is an acid having at least two acidic pKas. The other counterion is an acid with one or more pKas. Examples of suitable acids include hydrochloric acid, hydrobromic acid, nitric acid, sulfuric acid, maleic acid, phosphoric acid, benzene sulfonic acid, methane sulfonic acid, citric acid, succinic acid, glycolic acid, gluconic acid, glucuronic acid, lactic acid, malic acid, pyruvic acid, tartaric acid, tartronic acid, fumaric acid, acetic acid, propionic acid, pentanoic acid, carbonic acid, malonic acid, adipic acid, citraconic acid, levulinic acid, glutaric acid, itaconic acid, meglutol, mesaconic acid, citramalic acid, citric acid, aspartic acid, glutamic acid, tricarballylic acid and ethylenediaminetetraacetic acid.

Generally, in the noted embodiments of the invention, the amount of counterion should neutralize the charge of the biologically active agent. In such embodiments, the counterion or the mixture of counterion is present in amounts necessary to neutralize the charge present on the agent at the pH of the formulation. Excess of counterion (as the free acid or as a salt) can be added to the peptide in order to control pH and to provide adequate buffering capacity.

In one preferred embodiment, the agent has a positive charge and the counterion is a viscosity-enhancing mixture of counterions chosen from the group of citric acid, tartaric acid, malic acid, hydrochloric acid, glycolic acid, and acetic acid. Preferably, counterions are added to the formulation to achieve a viscosity in the range of about 2.10⁻² - 0,2 Pas (20 - 200 cp).

In a preferred embodiment, the viscosity enhancing counterion is an acidic counterion such as a low volatility weak acid. Low volatility weak acid counterions present at least one acidic pKa and a melting point higher than about 50°C or a boiling point higher than about 170° C at Pₐₜₘ. Examples of such acids include citric acid, succinic acid, glycolic acid, gluconic acid, glucuronic acid, lactic acid, malic acid, pyruvic acid, tartaric acid, tartronic acid, and fumaric acid.

In another preferred embodiment, the counterion is a strong acid. Strong acids can be defined as presenting at least one pKa lower than about 2. Examples of such acids include hydrochloric acid, hydrobromic acid, nitric acid, sulfonic acid, sulfuric acid, maleic acid, phosphoric acid, benzene sulfonic acid and methane sulfonic acid.

Another preferred embodiment is directed to a mixture of counterions wherein at least one of the counterion is a strong acid and at least one of the counterion is a low volatility weak acid.

Another preferred embodiment is directed to a mixture of counterions wherein at least one of the counterion is a strong acid and at least one of the counterion is a weak acid with high volatility. Volatile weak acid counterions present at least one pKa higher than about 2 and a melting point lower than about 50°C or a boiling point lower than about 170°C at Pₐₜₘ. Examples of such acids include acetic acid, propionic acid, pentanoic acid and the like.

The acidic counterion is present in amounts necessary to neutralize the positive charge present on the drug at the pH of the formulation. Excess of counterion (as the free acid or as a salt) can be added to the drug in order to control pH and to provide adequate buffering capacity.

In another embodiment of the invention, the coating formulation includes at least one buffer. Examples of such buffers include ascorbic acid, citric acid, succinic acid, glycolic acid, gluconic acid, glucuronic acid, lactic acid, malic acid, pyruvic acid, tartaric acid, tartronic acid, fumaric acid, maleic acid, phosphoric acid, tricarballylic acid, malonic acid, adipic acid, citraconic acid, glutaratic acid, itaconic acid, mesaconic acid, citramalic acid, dimethylolpropionic acid, tiglic acid, glyceric acid, methacrylic acid, isocrotonic acid, b-hydroxybutyric acid, crotonic acid, angelic acid, hydracrylic acid, aspartic acid, glutamic acid, glycine or mixtures thereof.

In one embodiment of the invention, the coating formulations include at least one antioxidant, which can be sequestering s such sodium citrate, citric acid, EDTA-(ethylene-dinitrilo-tetraacetic acid) or free radical scavengers such as ascorbic acid, methionine, sodium ascorbate, and the like.

In one embodiment of the invention, the coating formulation includes at least one surfactant, which can be zwitterionic, amphoteric, cationic, anionic, or nonionic, including, without limitation, sodium lauroamphoacetate, sodium dodecyl sulfate (SDS), cetylpyridinium chloride (CPC), dodecyltrimethyl ammonium chloride (TMAC), benzalkonium, chloride, polysorbates such as Tween 20 and Tween 80, other sorbitan derivatives, such as sorbitan laurate, and alkoxylated alcohols, such as laureth-4.

In a further embodiment of the invention, the coating formulation includes at least one polymeric material or polymer that has amphiphilic properties, which can comprise, without limitation, cellulose derivatives, such as hydroxyethylcellulose (HEC), hydroxypropylmethylcellulose (HPMC), hydroxypropycellulose (HPC), methylcellulose (MC), hydroxyethylmethylcellulose (HEMC), or ethylhydroxyethylcellulose (EHEC), as well as pluronics.

In another embodiment, the coating formulation includes a hydrophilic polymer selected from the following group: hydroxyethyl starch, dextran, poly(vinyl alcohol), poly(ethylene oxide), poly(2-hydroxyethylmethacrylate), poly(n-vinyl pyrolidone), polyethylene glycol and mixtures thereof , and like polymers.

In another embodiment of the invention, the coating formulation includes a biocompatible carrier, which can comprise, without limitation, human albumin, bioengineered human albumin, polyglutamic acid, polyaspartic acid, polyhistidine, pentosan polysulfate, polyamino acids, sucrose, trehalose, melezitose, raffinose and stachyose.

In another embodiment, the coating formulation includes a stabilizing agent, which can comprise, without limitation, a non-reducing sugar, a polysaccharide or a reducing sugar. Suitable non-reducing sugars for use in the methods and compositions of the invention include, for example, sucrose, trehalose, stachyose, or raffinose. Suitable polysaccharides-for use in the methods-and compositions of the invention include, for example, dextran, soluble starch, dextrin, and insulin. Suitable reducing sugars for use in the methods and compositions of the invention include, for example, monosaccharides such as, for example, apiose, arabinose, lyxose, ribose, xylose, digitoxose, fucose, quercitol, quinovose, rhamnose, allose, altrose, fructose, galactose, glucose, gulose, hamamelose, idose, mannose, tagatose, and the like; and disaccharides such as, for example, primeverose, vicianose, rutinose, scillabiose, cellobiose, gentiobiose, lactose, lactulose, maltose, melibiose, sophorose, and turanose, and the like.

In another embodiment, the coating formulation includes a vasoconstrictor, which can comprise, without limitation, amidephrine, cafaminol, cyclopentamine, deoxyepinephrine, epinephrine, felypressin, indanazoline, metizoline, midodrine, naphazoline, nordefrin, octodrine, ornipressin, oxymethazoline, phenylephrine, phenylethanolamine, phenylpropanolamine, propylhexedrine, pseudoephedrine, tetrahydrozoline, tramazoline, tuaminoheptane, tymazoline, vasopressin, xylometazoline and the mixtures thereof. The most preferred vasoconstrictors include epinephrine, naphazoline, tetrahydrozoline indanazoline, metizoline, tramazoline, tymazoline, oxymetazoline and xylometazoline.

As will be appreciated by one having ordinary skill in the art, the addition of a vasoconstrictor to the coating formulations and, hence, solid biocompatible coatings of the invention is particularly useful to prevent bleeding that can occur following application of the microprojection member or array and to prolong the pharmacokinetics of the biologically active agent through reduction of the blood flow at the application site and reduction of the absorption rate from the skin site into the system circulation.

In another embodiment of the invention, the coating formulation includes at least one "pathway patency modulator", which can comprise, without limitation, osmotic agents (e.g., sodium chloride), zwitterionic compounds (e.g., amino acids), and anti-inflammatory agents, such as betamethasone 21-phosphate disodium salt, triamcinolone acetonide 21-disodium phosphate, hydrocortamate hydrochloride, hydrocortisone 21-phosphate disodium salt, methylprednisolone 21-phosphate disodium salt, methylprednisolone 21-succinaate sodium salt, paramethasone disodium phosphate and prednisolone 21-succinate sodium salt, and anticoagulants, such as citric acid, citrate salts (e.g., sodium citrate), dextrin sulfate sodium, aspirin and EDTA.

In yet another embodiment of the invention, the coating formulation includes a solubilising/complexing agent, which can comprise Alpha-Cyclodextrin, Beta-Cyclodextrin, Gamma-Cyclodextrin, glucosyl-alpha-Cyclodextrin, maltosyl-alpha-Cyclodextrin, glucosyl-beta-Cyclodextrin, maltosyl-beta-Cyclodextrin, hydroxypropyl beta-cyclodextrin, 2-hydroxypropyl-beta-Cyclodextrin, 2-hydroxypropyl-gamma-Cyclodextrin, hydroxyethyl-beta-Cyclodextrin, methyl-beta-Cyclodextrin, sulfobutylether-alpha-cyclodextrin, sulfobutylether-beta-cyclodextrin, and sulfobutylether-gamma-cyclodextrin. Most preferred solubilising/complexing agents are beta-cyclodextrin, hydroxypropyl beta-cyclodextrin, 2-hydroxypropyl-beta-Cyclodextrin and sulfobutylether7 beta-cyclodextrin.

In another embodiment of the invention, the coating formulation includes at least one non-aqueous solvent, such as ethanol, isopropanol, methanol, propanol, butanol, propylene glycol, dimethysulfoxide, glycerin, N,N-dimethylformamide and polyethylene glycol 400.

Preferably, the coating formulations have a viscosity less than approximately 0,5 Pas (500 centipoise) and greater than 3.10⁻³ Pas (3 centipoise).

In one embodiment of the invention, the thickness of the biocompatible coating is less than 25 microns, more preferably, less than 10 microns, as measured from the microprojection surface.

The desired coating thickness is dependent-upon-several factors, including the required dosage and, hence, coating thickness necessary to deliver the dosage, the density of the microprojections per unit area of the sheet, the viscosity and concentration of the coating composition and the coating method chosen.

## Claims

1. A transdermal delivery device for delivering a biologically active agent comprising at least one stratum corneum-piercing microprojection (36), wherein said microprojection has a first portion with a hydrophobic coating and a second portion with a hydrophilic coating comprising said biologically active agent.

2. The device of claim 1, wherein said second portion comprises a distal portion of said microprojection.

3. The device of claim 1 or 2, further comprising multiple microprojections having said hydrophobic coating and said hydrophilic coating.

4. The device of claim 3, wherein said portion of each said microprojections comprises a distal portion of each said microprojections.

5. The device of claim 3 or 4, wherein said portion of each said microprojections comprises substantially the entire microprojection.

6. The device of any preceding claim, wherein said microprojection is configured to pierce through the stratum corneum to a depth of less than about 500 micrometers.

7. The device of any preceding claim, wherein said microprojection has a length of less than about 500 micrometers and a thickness of less than about 25. micrometers.

8. The device of claim 7, wherein said hydrophilic coating has a thickness equal to or less than the thickness of said microprojection.

9. The device of any preceding claim, wherein said stratum corneum-piercing microprojection is formed by etching said microprotrusion from a thin sheet and folding said microprojection out of a plane of the sheet.

10. The device of claim 9, where said thin sheet comprises a metallic material.

11. The device of claim 10, wherein said metallic material is selected from the group consisting of stainless steel, titanium and nickel titanium alloys.

12. The device of any preceding claim, wherein said agent is selected from the group consisting of growth hormone release hormone (GHRH), growth hormone release factor (GHRF), insulin, insultropin, calcitonin, octreotide, endorphin, TRN, NT-36 (chemical name: N-[[(s)-4-oxo-2-azetidinyl] carbonyl]-L-histidyl-L-prolinamide), liprecin, pituitary hormones (e.g., HGH, HMG, desmopressin acetate, etc), follicle luteoids, aANF, growth factors such as growth factor releasing factor (GFRF), bMSH, GH, somatostatin, bradykinin, somatotropin, platelet-derived growth factor releasing factor, asparaginase, bleomycin sulfate, chymopapain, cholecystokinin, chorionic gonadotropin, erythropoietin, epoprostenol (platelet aggregation inhibitor), gluagon, HCG, hirulog, hyaluronidase, interferon alpha, interferon beta, interferon gamma, interleukins, interleukin-10 (IL-10), erythropoietin (EPO), granulocyte macrophage colony stimulating factor (GM-CSF), granulocyte colony stimulating factor (G-CSF), glucagon, leutinizing hormone releasing hormone (LHRH), LHRH analogs (such as goserelin, leuprolide, buserelin, triptorelin, gonadorelin, and napfarelin, menotropins (urofollitropin (FSH) and LH)), oxytocin, streptokinase, tissue plasminogen activator, urokinase, vasopressin, deamino [Val4, D-Arg8] arginine vasopressin, desmopressin, corticotropin (ACTH), ACTH analogs such as ACTH (1-24), ANP, ANP clearance inhibitors, angiotensin II antagonists, antidiuretic hormone agonists, bradykinn antagonists, ceredase, CSI's, calcitonin gene related peptide (CGRP), enkephalins, FAB fragments, IgE peptide suppressors, IGF-1, neurotrophic factors, colony stimulating factors, parathyroid hormone and agonists, parathyroid hormone antagonists, parathyroid hormone (PTH), PTH analogs such as PTH (1-34), prostaglandin antagonists, pentigetide, protein C, protein S, renin inhibitors, thymosin alpha-1, thrombolytics, TNF, vasopressin antagonists analogs, alpha-1 antitrypsin (recombinant), and TGF-beta.

13. The device of any preceding claim, wherein said hydrophobic coating is disposed on a layer of photoresist.

14. The device of any preceding claim, wherein said hydrophobic coating is disposed on a portion of said microprojection having been washed free of unexposed photoresist.

15. The device of claim 14, wherein said hydrophilic coating is disposed on a portion of said microprojection having been washed free of solubilized exposed photoresist.

16. The device of any preceding claim, wherein said hydrophilic coating includes a further component selected from the viscosity enhancing counterions group consisting of:
an antioxidant;
an amphiphilic polymer;
a surfactant;
a hydrophilic polymer;
a biocompatible carrier;
a stabilizing agent;
a vasoconstrictor;
a pathway patency modulator; and
a solubilising/complexing agent.

17. The device of any preceding claim, wherein said hydrophilic coating has a viscosity less than about 0,5 Pas (500 centipoise).

18. The device of any one of claims 3 to 17, wherein the microprojection density is at least about 10 microprojections/cm².

19. The device of claim 18, wherein the microprojection density is about 200 - 2000 microprojections/cm².

20. A method for forming a device for transdermally delivering a biologically active agent comprising the steps of:
forming at least one stratum corneum-piercing microprojection (36) in a thin sheet of material;
applying a hydrophobic coating to a first portion of said microprojection; and
applying a hydrophilic coating comprising said biologically active agent to a second portion of said microprojection.

21. The method of claim 20, further comprising the step of bending said microprojection out of a plane formed by said thin sheet after applying said hydrophobic coating.

22. The method of claim 20 or 21, wherein the step of forming said microprojection is selected from the group consisting of etching and punching.

23. The method of claim 20 or 21, wherein the step of forming said microprojection comprises etching.

24. The method of any one of claims 20 to 23, further comprising the step of removing a hydrophobic coating from said second portion of said microprojection member before applying said hydrophilic coating.

25. The method of claim 24, wherein the step of removing a hydrophobic coating from said second portion of said microprojection member comprises vaporizing said hydrophobic coating with radiation.

26. The method of claim 24, wherein the step of removing a hydrophobic coating from said second portion of said microprojection member comprises contacting said second portion with a micro-stamp.

27. The method of any one of claims 20 to 26, wherein the step of applying a hydrophobic coating to a first portion of said microprojection comprises:
coating said microprojection with photoresist;
masking said second portion of said microprojection;
exposing said microprojection to radiation;
washing unexposed photoresist from said first portion of said microprojection member;
applying said hydrophobic coating to said first and second portions of said microprojection;
solubilizing exposed photoresist on said second portion of said microprojection; and
washing said solubilized exposed photoresist from said second portion of said microprojection.

28. The method of any one of claims 20 to 26, wherein the step of applying a hydrophobic coating to a first portion of said microprojection comprises contacting said first portion with a micro-stamp.

## Patentansprüche

1. Transdermale Abgabevorrichtung zur Abgabe eines biologisch aktiven Mittels umfassend wenigstens eine Stratum Corneum-Piercing-Mikroprojektion (36), bei der die Mikroprojektion einen ersten Abschnitt mit einer hydrophoben Beschichtung und einen zweiten Abschnitt mit einer hydrophilen Beschichtung, die das biologisch aktive Mittel aufweist, hat.

2. Vorrichtung nach Anspruch 1, bei der zweite Abschnitt einen Distalabschnitt der Mikroprojektion aufweist.

3. Vorrichtung nach Anspruch 1 oder 2, die des weiteren mehrere Mikroprojektionen mit der hydrophoben Beschichtung und der hydrophilen Beschichtung aufweist.

4. Vorrichtung nach Anspruch 3, bei der der Abschnitt jeder der Mikroprojektionen einen Distalabschnitt von jeder Mikroprojektion umfaßt.

5. Vorrichtung nach Anspruch 3 oder 4, bei der der Abschnitt jeder der Mikroprojektionen im wesentlichen die gesamte Mikroprojektion umfaßt.

6. Vorrichtung nach einem der vorherigen Ansprüche, bei dem die Mikroprojektion so konfiguriert ist, um durch das Stratum Corneum mit einer Tiefe von weniger als etwa 500 Mikrometer zu stechen.

7. Vorrichtung nach einem der vorherigen Ansprüche, bei der die Mikroprojektion eine Länge von weniger als etwa 500 Mikrometer und eine Dicke von weniger als etwa 25 Mikrometer hat.

8. Vorrichtung nach Anspruch 7, bei der die hydrophile Beschichtung eine Dicke hat, die gleich oder geringer als die Dicke der Mikroprojetion ist.

9. Vorrichtung nach einem der vorherigen Ansprüche, bei dem die Stratum Corneum-Piercing-Mikroprojektion durch Ätzen des Mikrovorsprungs von einer dünnen Folie und Falten der Mikroprojektion aus der Ebene der Folie heraus gebildet wird.

10. Vorrichtung nach Anspruch 9, bei der die dünne Folie ein metallisches Material aufweist.

11. Vorrichtung nach Anspruch 10, bei der das metallische Material ausgewählt wird aus der Gruppe bestehend aus rostfreiem Stahl, Titan und Nickel-Titan-Legierungen.

12. Vorrichtung nach einem der vorherigen Ansprüche, bei der das Mittel ausgewählt wird aus der Gruppe bestehend aus Wachstumhormon-Releasehormon (GHRH), Wachstumshormon-Reteasefaktor (GHRF), Insulin, Insultropin, Calcitonin, Octreotid, Endorphin, TRN, NT-36 (chemische Bezeichnung: N-[[(s)-4-Oxo-2-azetidinyl]carbonyl]-L-histidyl-L-prolinamid), Liprecin, Hyperphysenhormone (z.B., HGH, HMG, Desmopressinacetat, usw.), Follikel-Luteoide, aANF, Wachstumsfaktoren so wie Wachstumsfaktor-Releasing-Faktor (GFRF), BMSH, GH, Somatostatin. Bradykinin, Somatotropin, Piateiet-denved Growth Factor Releasing Factor, Asparaginase, Bleomycinsulfat, Chymopapain, Cholecystokinin, Chorionisches Gonadotropin, Erythropoietin, Epoprostenol (platelet aggregation inhibitor). Gluagon, HCG, Hirulog, Hyaluronidase, Interferon alpha, Interferon beta, Interferon gamma, Interleukine, Interleukin-10 (IL-10), Erythropoietin (EPO), granulocyte macrophage colony stimulating factor (GM-CSF), granulocyte colony stimulating factor (G-CSF), Glukagon, luteinizing hormone releasing hormone (LHRH), LHRH-Analoge (wie Goserelin, Leuprolid, Buserelin, Triptorelin, Gonadorelin und Napfarelin, Menotropine (Urofollitropin (FSH) und LH)), Oxytocin, Streptokinase, tissue plasminogen activator, Urokinase, Vasopression, Deamino[Val4, D-Arg8]argininvasopressin, Desmopressin, Corticotropin (ACTH), ACTH-Analoge wie ACTH (1-24), ANP, ANP clearance inhibitors, Angiotensin II Antagonisten, Antidiuretische Hormon-Agonisten, Bradykinin Antagonisten, Ceredase, CSI's, calcitonin gene related peptide (CGRP), Enkephaline, FAB Fragmente, IgE Peptid Suppressoren, IGF-1, neurotrophe Faktoren, colony stimulating factors, Parathyroid Hormone und Agonisten, Parathyroid Hormon Antagonisten, Parathyroid Hormon (PTH), PTH-Analoge wie PTH (1-34), Prostaglandin Antagonisten, Pentigetid, Protein C, Protein S, Renin Inhibitoren, Thymosin alpha-1, Thrombolyten, TNF, Vasopressin Antagonisten Analoge, alpha-1 Antitrypsin (rekombinant), und TGF-beta.

13. Vorrichtung nach einem der vorherigen Ansprüche, bei der die hydrophobe Beschichtung auf einer Schicht eines Photoresist angeordnet ist.

14. Vorrichtung nach einem der vorherigen Ansprüche, bei der die hydrophobe Beschichtung auf einem Abschnitt der Mikroprojektion angeordnet ist, von der der unbelichtete Photoresist abgewaschen wird.

15. Vorrichtung nach Anspruch 15, bei der die hydrophile Beschichtung auf einem Abschnitt der Mikroprojektion angeordnet ist, von der der lösliche belichtete Photoorestist abgewaschen wird.

16. Vorrichtung nach einem der vorherigen Ansprüche, bei der die hydrophile Beschichtung eine weiteren Bestandteil einschließt, der ausgewählt wird aus der viskositätsverstärkender Gegeniongruppe bestehend aus:
einem Antioxidans;
einem amphiphilen Polymer;
einem Tensid;
einem hydrophilen Polymer;
einem biokompatiblen Träger;
einem Stabilisierungsmittel;
einem Vasokonstriktor,
einem Pathway-Durchgängigkeitsmodulator; und
einem lösungsvermittelndem/komplexbildenden Mittel.

17. Vorrichtung nach einem der vorherigen Ansprüche, bei der die hydrophile Beschichtung einen Viskosität von weniger als etwa 0,5 Pas (500 Centipoise) aufweist.

18. Vorrichtung nach einem der Ansprüche 3 bis 17, bei der die Mikroprojektiondichte weniger als etwa 10 Mikroprojektionen/cm² ist.

19. Vorrichtung nach Anspruch 18, bei der die Mikroprojektiondichte etwa 200 bis 2000 Mikroprojektionen/cm² ist.

20. Verfahren zur Herstellung einer Vorrichtung zur transdermalen Abgabe eines biologisch aktiven Mittels, umfassend die Schritte:
Bildung wenigstens einer Stratum Corneum-Piercing-Mikroprojektion (36) in einer dünnen Folie eines Materials;
Auftragen einer hydrophoben Beschichtung auf einen ersten Abschnitt der Mikroprojektion: und
Auftragen einer hydrophilen Beschichtung umfassend das biologisch aktive Mittel auf einen zweiten Abschnitt der Mikroprojektion.

21. Verfahren nach Anspruch 20, weiterhin umfassend den Schritt des Biegens der Mikroprojektion aus der Ebene heraus, die durch die dünne Folie nach Auftragen der hydrophoben Beschichtung gebildet wird.

22. Verfahren nach Anspruch 20 oder 21, bei dem der Schritt der Bildung der Mikroprojektion ausgewählt wird aus der Gruppe bestehend aus Ätzen und Stanzen.

23. Verfahren nach Anspruch 20 oder 21, bei dem der Schritt der Bildung der Mikroprojektion Ätzen umfaßt.

24. Verfahren nach einem der Ansprüche 20 bis 23, weiterhin umfassend den Schritt der Entfernung einer hydrophoben Beschichtung von dem zweiten Abschnitt des Mikroprojektionselement bevor die hydrophile Beschichtung aufgetragen wird.

25. Verfahren nach Anspruch 24, bei dem der Schritt der Entfernung einer hydrophoben Beschichtung von dem zweiten Abschnitt des Mikroprojektionselement das Verdampfen der hydrophoben Beschichtung mit Bestrahlung umfaßt.

26. Verfahren nach Anspruch 24, bei der Schritt der Entfernung eine hydrophoben Beschichtung von dem zweiten Abschnitt des Mikroprojektionselements das Inkontrakt bringen des zweiten Abschnitts mit einem Mikrostempel umfaßt.

27. Verfahren nach einem der Ansprüche 20 bis 26, bei dem der Schritt des Auftragen eine hydrophoben Beschichtung auf einem ersten Abschnitt der Mikroprojektion umfaßt:
Beschichten der Mikroprojektion mit Photoresist;
Maskieren des zweiten Abschnitts der Mikroprojektion;
Aussetzen der Mikroprojektion gegenüber Bestrahlung;
Abwaschen des unbelichteten Photoresist von dem ersten Abschnitt des Mikroprojektionselements;
Auftragen der hydrophoben Beschichtung auf die ersten und zweiten Abschnitte der Mikroprojektion;
Ablösen von belichtetem Photoresist von dem zweiten Abschnitt der Mikroprojektion; und
Abwaschen des gelösten belichteten Photoresists von dem zweiten Abschnitt der Mikroprojektion.

28. Verfahren nach einem der Ansprüche 20 bis 26. bei dem der Schritt des Auftragens einer hydrophoben Beschichtung auf einen ersten Abschnitt der Mikroprojektion das Inkontakt bringen des ersten Abschnitts mit einem Mikrostempel umfaßt.

## Revendications

1. - Dispositif d'administration transdermique pour administrer un agent biologiquement actif comprenant au moins une microprojection (36) perçant le stratum corneum, ladite microprojection ayant une première partie avec un revêtement hydrophobe et une seconde partie avec un revêtement hydrophile comprenant ledit agent biologiquement actif.

2. - Dispositif selon la revendication 1, dans lequel ladite seconde partie comprend une partie distale de ladite microprojection.

3. - Dispositif selon l'une des revendications 1 ou 2, comprenant en outre de multiples microprojections ayant ledit revêtement hydrophobe et ledit revêtement hydrophile.

4. - Dispositif selon la revendication 3, dans lequel ladite partie de chacune desdites microprojections comprend une partie distale de chacune desdites microprojections.

5. - Dispositif selon l'une des revendications 3 ou 4, dans lequel ladite partie de chacune desdites microprojections comprend sensiblement la microprojection entière.

6. - Dispositif selon l'une quelconque des revendications précédentes, dans lequel ladite microprojection est configurée pour percer à travers le stratum corneum jusqu'à une profondeur inférieure à environ 500 micromètres.

7. - Dispositif selon l'une quelconque des revendications précédentes, dans lequel ladite microprojection a une longueur inférieure à environ 500 micromètres et une épaisseur inférieure à environ 25 micromètres.

8. - Dispositif selon la revendication 7, dans lequel ledit revêtement hydrophile a une épaisseur inférieure ou égale à l'épaisseur de ladite microprojection.

9. - Dispositif selon l'une quelconque des revendications précédentes, dans lequel ladite microprojection perçant le stratum corneum est formée par attaque chimique de ladite microprotubérance à partir d'une feuille mince et par pliage de ladite microprojection hors d'un plan de la feuille.

10. - Dispositif selon la revendication 9, dans lequel ladite feuille mince comprend une matière métallique.

11. - Dispositif selon la revendication 10, dans lequel ladite matière métallique est choisie dans le groupe constitué par l'acier inoxydable, le titane et les alliages nickel-titane.

12. - Dispositif selon l'une quelconque des revendications précédentes, dans lequel ledit agent est choisi dans le groupe constitué par l'hormone de libération de l'hormone de croissance (GHRH), le facteur de libération de l'hormone de croissance (GHRF), l'insuline, l'insulinotropine, la calcitonine, l'octréotide, l'endorphine, TRN, NT-36 (nom chimique : N-[[(s)-4-oxo-2-azétidinyl]carbonyl]-L-histidyl-L-prolinamide), la liprécine, les hormones pituitaires (par exemple, HGH, HMG, l'acétate de desmopressine, etc), les hormones lutéiniques folliculaires, aANF, les facteurs de croissance tels que le facteur de libération du facteur de croissance (GFRF), bMSH, GH, la somatostatine, la bradykinine, la somatotropine, le facteur de libération du facteur de croissance dérivé des plaquettes, l'asparaginase, le sulfate de bléomycine, la chymopapaïne, la cholécystokinine, la gonadotrophine chorionique, l'érythropoïétine, l'époprosténol (inhibiteur de l'agrégation plaquettaire), le glucagon, HCG, l'hirulog, la hyaluronidase, l'interféron alpha, l'interféron bêta, l'interféron gamma, les interleukines, l'interleukine-10 (IL-10), l'érythropoïétine (EPO), le facteur de stimulation des colonies de granulocytes-macrophages (GM-CSF), le facteur de stimulation des colonies de granulocytes (G-CSF), le glucagon, l'hormone de libération de l'hormone lutéinisante (LHRH), les analogues de la LHRH (tels que la goséréline, le leuprolide, la buséréline, la triptoréline, la gonadoréline et la napfaréline), les ménotropines (urofollitropine (FSH) et (LH)), l'oxytocine, la streptokinase, l'activateur tissulaire du plasminogène, l'urokinase, la vasopressine, la désamino[Val4, D-Arg8]arginine vasopressine, la desmopressine, la corticotropine (ACTH), les analogues de la ACTH tels que ACTH (1-24), ANP, les inhibiteurs de la clairance de l'ANP, les antagonistes de l'angiotensine II, les agonistes de l'hormone antidiurétique, les antagonistes de la bradykinine, la cérédase, les CSI, le peptide apparenté au gène de la calcitonine (CGRP), les encéphalines, les fragments FAB, les suppresseurs de peptide IgE, IGF-1, les facteurs neurotrophiques, les facteurs de stimulation des colonies, l'hormone parathyroïdienne et les agonistes, les antagonistes de l'hormone parathyroïdienne, l'hormone parathyroïdienne (PTH), les analogues de PTH tels que PTH (1-34), les antagonistes de la prostaglandine, le pentigétide, la protéine C, la protéine S, les inhibiteurs de la rénine, la thymosine alpha-1, les thrombolytiques, TNF, les analogues des antagonistes de la vasopressine, l'antitrypsine alpha-1 (recombinante) et le TGF-bêta.

13. - Dispositif selon l'une quelconque des revendications précédentes, dans lequel ledit revêtement hydrophobe est disposé sur une couche de photorésist.

14. - Dispositif selon l'une quelconque des revendications précédentes, dans lequel ledit revêtement hydrophobe est disposé sur une partie de ladite microprojection ayant été lavée pour être débarrassée du photorésist non exposé.

15. - Dispositif selon la revendication 14, dans lequel ledit revêtement hydrophile est disposé sur une partie de ladite microprojection ayant été lavée pour être débarrassée du photorésist exposé solubilisé.

16. - Dispositif selon l'une quelconque des revendications précédentes, dans lequel ledit revêtement hydrophile comprend un autre composant choisi dans le groupe des contre-ions améliorant la viscosité constitué de :
- un antioxydant ;
- un polymère amphiphile ;
- un agent tensio-actif ;
- un polymère hydrophile ;
- un support biocompatible ;
- un agent stabilisant ;
- un vasoconstricteur ;
- un modulateur de perméabilité de la voie de passage : et
- un agent solubilisant/complexant.

17. - Dispositif selon l'une quelconque des revendications précédentes, dans lequel ledit revêtement hydrophile a une viscosité inférieure à environ 0,5 Pa.s (500 centipoises).

18. - Dispositif selon l'une quelconque des revendications 3 à 17, dans lequel la densité des microprojections est au moins d'environ 10 microprojections/cm².

19. - Dispositif selon la revendication 18, dans lequel la densité des microprojections est d'environ 200-2000 microprojections/cm².

20. - Procédé pour former un dispositif d'administration transdermique d'un agent biologiquement actif comprenant les étapes consistant à :
- former au moins une microprojection (36) perçant le stratum corneum dans une feuille mince de matière ;
- appliquer un revêtement hydrophobe sur une première partie de ladite microprojection ; et
- appliquer un revêtement hydrophile comprenant ledit agent biologiquement actif sur une seconde partie de ladite microprojection.

21. - Procédé selon la revendication 20, comprenant en outre l'étape consistant à plier ladite microprojection hors d'un plan formé par ladite feuille mince après avoir appliqué ledit revêtement hydrophobe.

22. - Procédé selon l'une des revendications 20 ou 21, dans lequel l'étape de formation de ladite microprojection est choisie dans le groupe constitué par l'attaque chimique et le poinçonnage.

23. - Procédé selon l'une des revendications 20 ou 21, dans lequel l'étape de formation de ladite microprojection comprend l'attaque chimique.

24. - Procédé selon l'une quelconque des revendications 20 à 23, comprenant en outre l'étape de retrait d'un revêtement hydrophobe à partir de ladite seconde partie dudit élément de microprojection avant l'application dudit revêtement hydrophile.

25. - Procédé selon la revendication 24, dans lequel l'étape de retrait d'un revêtement hydrophobe à partir de ladite seconde partie dudit élément de microprojection comprend la vaporisation dudit revêtement hydrophobe avec radiation.

26. - Procédé selon la revendication 24, dans lequel l'étape de retrait d'un revêtement hydrophobe à partir de ladite seconde partie dudit élément de microprojection comprend la mise en contact de ladite seconde partie avec un micro-poinçon.

27. - Procédé selon l'une quelconque des revendications 20 à 26, dans lequel l'étape d'application d'un revêtement hydrophobe sur une première partie de ladite microprojection comprend les opérations consistant à :
- revêtir ladite microprojection avec le photorésist ;
- masquer ladite seconde partie de ladite microprojection ;
- exposer ladite microprojection à une radiation ;
- laver le photorésist non exposé à partir de ladite première partie dudit élément de microprojection ;
- appliquer ledit revêtement hydrophobe sur lesdites première et seconde parties de ladite microprojection ;
- solubiliser le photorésist exposé sur ladite seconde partie de ladite microprojection ; et
- laver ledit photorésist exposé solubilisé à partir de ladite seconde partie de ladite microprojection.

28. - Procédé selon l'une quelconque des revendications 20 à 26, dans lequel l'étape d'application d'un revêtement hydrophobe sur une première partie de ladite microprojection comprend la mise en contact de ladite première partie avec un micro-poinçon.
